(19) Europäisches Patentamt
European Patent Office
Office européen des brevets

(11) Publication number: **0 003 242**
**B1**

(12) **EUROPEAN PATENT SPECIFICATION**

(45) Date of publication of patent specification: **20.01.82**

(51) Int. Cl.³: **C 07 C 121/75,**
C 07 C 121/66

(21) Application number: **78200349.5**

(22) Date of filing: **06.12.78**

(54) **Process for the preparation of alpha-cyanobenzyl esters.**

(30) Priority: **14.12.77 US 860507**

(43) Date of publication of application:
**08.08.79 Bulletin 79/16**

(45) Publication of the grant of the European patent:
**20.01.82 Bulletin 82/3**

(84) Designated Contracting States:
**BE CH DE FR GB IT LU NL**

(56) References cited:
**FR - A - 2 342 960**

**D. J. CRAM et al "Organic Chemistry" 1959, MC Graw-Hill, New York * page 308, paragraph 2; page 507 paragraph 3, page 508, paragraph 1 ***

**S. COFFEY "Rodd's chemistry of carbon compounds" 2nd edition, vol. I, part F: "Penta- and higher polyhydric alcohols, their oxidation products and derivatives, saccharides 1967, Elsevier, Amsterdam * Page 381, paragraph 3**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Tieman, Charles Henry**
**2209 Freemont Street**
**Modesto California, 95350 (US)**

(74) Representative: **Keuzenkamp, Abraham et al,**
**P.O. Box 302**
**NL-2501 CH Den Haag (NL)**

(56) References cited:
**S. PATAI "The chemistry of acylhalides" 1972 INTERSCIENCE PUBLISHERS, London * page 203, paragraph 2**

**EP 0 003 242 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of alpha-cyanobenzyl esters

This invention relates to a process for the preparation of *alpha*-cyanobenzyl esters. These esters belong to a class of insecticidally-active compounds commonly referred to as synthetic pyrethroids.

It is known to prepare esters by the reaction of an acid halide with an alcohol — see for example "Organic Chemistry" (D. G. Cram et al, 1959, page 308, paragraph 2). It is also known from French Patent 2.342.960 that alpha-cyanobenzyl esters can be prepared by the reaction of a benzaldehyde with an acid chloride in the presence of an alkali metal or ammonium cyanide and a phase transfer catalyst. The Applicant has found a valuable new method of producing alpha-cyanobenzyl esters which gives rise to excellent yields of the said esters.

The present invention provides a process for the preparation of *alpha*-cyanobenzyl esters of general formula I:

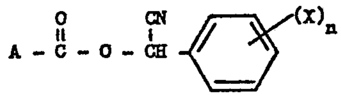
(I)

by reacting a carboxylic acid halide of general formula II:—

A—CO-halide (II)

with an *alpha*-cyanobenzyl alcohol of general formula III:—

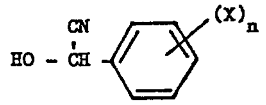
(III)

in the presence of an inert solvent, wherein in the general formulae A is an optionally-substituted aralkyl, alkyl or cycloalkyl group containing up to 24 carbon atoms; X is an alkyl, alkenyl, aralkyl or aryloxy group containing up to 10 carbon atoms; n is an integer of 1 to 3; and halide is bromine or chlorine, characterised in that the reaction is carried out in the presence of a Lewis acid catalyst. Preferably, the reaction is conducted in the presence of an inert solvent under refluxing conditions.

Preferably, n is 1 and X is in the 3-position relative to the benzyl carbon atom. Generally speaking, X is an alkyl group containing 1 to 6 carbon atoms, especially methyl, an alkenyl group containing 2 to 4 carbon atoms, such as allyl, or an aralkyl or aryloxy group preferably containing up to 8 carbon atoms, such as benzyl or especially phenoxy. The most preferred *alpha*-cyanobenzyl alcohol of general formula (III) is *alpha*-cyano-3-phenoxybenzyl alcohol.

When A represents an optionally-substituted aralkyl group in formula (I), preferred compounds are those containing a benzyl group of formula (IV):

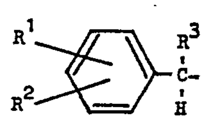
(IV)

wherein $R^3$ is an alkyl, cycloalkyl or alkenyl group containing up to 4 carbon atoms and $R^1$ is a hydrogen atom, a halogen atom having an atomic number of from 9 to 53, inclusive, $NO_2$, $CF_3$, an alkyl group containing 1 to 4 carbon atoms, an alkoxy group containing 1 or 2 carbon atoms, an allyl group or a propargyl group, and $R^2$ is a hydrogen atom or a methyl group.

Preferred because of their pesticidal properties are those pyrethroids wherein A is a benzyl group of formula (IV), in which $R^3$ is a branched-chain alkyl group containing up to 4 carbon atoms, such as isopropyl, $R^1$ is a halogen atom or an alkyl group containing 1 to 4 carbon atoms or alkoxy group containing 1 or 2 carbon atoms, for example, a chlorine atom, methyl or methoxy and $R^2$ is a hydrogen atom.

The most preferred benzyl-carboxylic acid halide starting material is *alpha*-isopropyl-4-chlorophenylacetyl chloride.

When A represents an optionally substituted cycloalkyl group in formula (I), the preferred compounds are those containing a cyclopropyl group of formula (V):

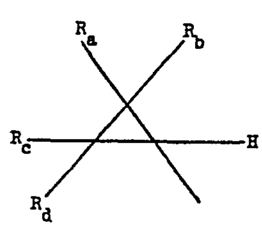
(V)

wherein $R_a$ and $R_b$ each represent an alkyl group having from 1 to 6 carbon atoms, especially methyl, a halogen atom having an atomic number of from 9 to 35, inclusive, especially a chlorine atom, or when $R_a$ represents a hydrogen atom then $R_b$ represents an alkynyl group having from 2 to 6 carbon atoms optionally substituted by from 1 to 3 fluorine, chlorine and/or bromine atoms, especially an isobutenyl, a mono- or dichlorovinyl or dibromovinyl group; $R_c$ and $R_d$ each represent an alkyl group having 1 to 6 carbon atoms, or when $R_c$ is hydrogen then $R_d$ is an alkenyl group having 2 to 6 carbon atoms optionally substituted by from 1 to 3 fluorine, chlorine and/or bromine atoms, or at least one of $R_a$ and $R_b$ together or $R_c$ and $R_d$ together each represent an alkylene group having from 2 to 6, especially 3, carbon atoms.

The most preferred cyclopropyl acid halide starting material is 2,2-dimethyl-3-(2,2-dibromovinyl)cyclopropanecarbonyl chloride, 2,2-dimethyl-3-(2,2-dichlorovinyl)cyclopropanecarbonyl chloride, 2,2,3,3-tetramethylcyclopropanecarbonyl chloride, or 2,2-dimethyl-3-isobutenylcyclopropanecarbonyl chloride.

The pyrethroid compounds prepared by the process according to the invention are known as pesticides, see for example, when A is a cyclopropyl group of formula (II), U.S. patents 3,835,176 and 3,987,193, Netherlands patent applications 7307130, 7212973 and 7205298, Belgian patents 814,819 and 820,418, German patent application 2,407,024. When A is an optionally substituted benzyl group of formula (IV), examples of pyrethroids may be found in Belgian patent 801,946; or when A is an alkyl group: Belgian patent 842,061.

The most preferred *alpha*-cyanobenzyl pyrethroids for use in pesticidal compositions have the formula (I), wherein A is *alpha*-isopropyl-4-chlorobenzyl, 2,2,3,3-tetramethylcyclopropyl, 2-(2,2-dichlorovinyl)-3,3-dimethylcyclopropyl, or 2-(2,2-dibromovinyl)-3,3-dimethylcyclopropyl, X is 3-phenoxy and n is 1.

It should be noted that the compounds prepared by the process according to the present invention can exist in the form of various stereoisomers by virtue of the existence of asymmetric carbon atoms in their structure, and thus racemates, single isomers, or isomer mixtures can be prepared by the process according to the invention by employing the appropriate starting materials.

It has been shown that in the case of *alpha*-cyano-3-phenoxybenzyl *alpha*-isopropylphenylacetates, such as *alpha*-cyano-3-phenoxybenzyl *alpha*-isopropyl-4-chlorophenylacetate, the process of the present invention provides not only improved chemical purity of the product but an improvement in the proportion of ester derived from the (+)-S- acid and the (—)-S-cyanobenzyl alcohol. That is, the product ester contains unexpectedly more than 50% of the enantiomeric pair containing the (+)-S-acid and (—)-S-cyanobenzyl alcohol ester. Usually this is about 55%.

In the case of the pyrethoids of formula (I) in which A is optionally substituted benzyl, it is known that the ester of the (+)-S-acid and the (—)-S-cyanobenzyl alcohol tend to be the most pesticidally active of the various isomers and are preferred for that reason. It follows, therefore, that it is desirable to prepare more of the ester derived from the (+)-S-acid and (—)-S-cyanobenzyl alcohol.

The catalyst may be any Lewis acid, that is, a compound which will accept one or more pairs of electrons from another compound to form a co-ordinate covalent bond. Suitably, the Lewis acid is derived from an element of Group IIIA of the Periodic Table of Elements (Lange, Handbook of Chemistry, 8th Edition, pages 56—57) titanium, tin, antimony, tantalum, rhenium, iron or zinc. These include inorganic compounds, derived from boron, aluminium, gallium, indium, thallium, zinc, titanium, antimony, iron and the like. Generally, the inorganic Lewis acid halides and cyanides are preferred. These include boron trichloride, boron tribromide, aluminium chloride, aluminium bromide, gallium trichloride, gallium tribromide, titanium tetrachloride, titanium tetrabromide, stannic chloride, stannic bromide, antimony pentachloride, tantalum pentachloride, rhenium pentachloride, ferric chloride and especially zinc salts, such as zinc chloride, zinc iodide or zinc cyanide.

Optimum amounts of Lewis acid depend on the particular catalyst chosen, the solvent and the reaction components as well as the reaction time and product purity desired. Usually the molar ratio of catalyst to *alpha*-cyanobenzyl alcohol is from 1:5 to 1:500, preferably 1:10 to 1:100.

The solvent should be inert to the reaction. Suitable solvents include alkane, cycloalkane and aromatic hydrocarbons, chlorinated hydrocarbons or ethers and mixtures thereof. More particularly the solvent is an alkane containing 5 to 10 carbon atoms, such as n-pentane, n-hexane, n-octane, n-heptane, n-nonane, n-decane and their isomers. Gasolines rich in alkanes are also very suitable, for example, with a boiling range at atmospheric pressure of between 40 and 65°C, 60 to 80°C or 80 to 110°C. Petroleum ether is also suitable. Cyclohexane and methylcyclohexane are examples of cycloalkanes containing 6 to 8 carbon atoms. Aromatic hydrocarbon solvents can contain from 6 to 10 carbon atoms, for example, benzene, toluene, o-, m- and p-xylene, the trimethylbenzenes, p-ethyltoluene and the like. Suitable chlorinated hydrocarbons contain from 1 to 4 chlorine atoms in combination with an alkane chain containing from 1 to 4 carbon atoms or a benzene ring, for example, carbon tetrachloride, chloroform, dichloromethane, 1,2-dichloroethane, trichloroethane, perchloroethane, chlorobenzene and 1,2- and 1,3-dichlorobenzene. Ethers are generally those containing 2 to 6 carbon atoms, such as diethyl ether and diisopropyl ether. Chlorinated hydrocarbons are preferred, especially methyl halides, such as methylene chloride.

The molar ratio of the acid halide to *alpha*-cyanobenzyl alcohol is not critical and can be varied, e.g., from 1:3 to 3:1 and preferably from 1.5 to 1 to 1 to 1.5.

The preferred reaction temperature is such as to constitute refluxing conditions if at normal atmospheric pressure and will naturally vary as to the nature of the composition of the reaction mixture.

The various ingredients of the reaction mixture are contacted, preferably with agitation, e.g., stirring. The acid halide may also be gradually added to a mixture of the other starting compound and reaction ingredients.

Conventionally, after a Lewis acid catalyzed reaction has been completed, or has been carried out to a desired degree of conversion, it can be advantageous to inactivate the Lewis acid before any components of the reaction mixture are isolated. This inactivation of the Lewis acid can be conveniently accomplished by extraction of the reaction mixture with water.

The products of the process of the invention are then recovered by suitable techniques of filtration, distillation and the like.

The following Examples are provided for the purpose of illustrating the invention. The identity of the products was confirmed by GLC, infrared and nuclear magnetic resonance spectral analysis as necessary.

EXAMPLE 1

A solution of 10.0 ml of 4-chloro-*alpha*-isopropylphenylacetyl chloride, 11.3 g of *alpha*-cyano-3-phenoxybenzyl alcohol and 0.2 g of zinc chloride in 50 ml of methylene chloride was stirred and refluxed for three hours. The solution was cooled to room temperature and stirred with aqueous sodium bicarbonate for one hour. The organic phase was separated, dried with magnesium sulphate and evaporated at reduced pressure to afford 20.0 g (95% yield) of the desired product of 95% purity by GLC analysis.

EXAMPLE 2

A procedure similar to that of Example 1, but using zinc iodide as a catalyst, gave 91% yield of the desired ester product of 92.4% purity by GLC analysis.

EXAMPLE 3

A reaction similar to Example 1 using zinc cyanide as a catalyst gave 100% yield of the desired ester product of 80% purity by GLC analysis.

Following procedures similar to those set forth in the Examples above, *alpha*-cyano-3-phenoxybenzyl 2,2-dimethyl-3-(2,2-dibromovinyl)cyclopropanecarboxylate, *alpha*-cyano-3-phenoxybenzyl 2,2-dimethyl-3-(2,2-dichlorovinyl)cyclopropanecarboxylate and *alpha*-cyano-3-phenoxybenzyl 2,2-dimethyl-3-isobutenylcyclopropanecarboxylate are obtained.

**Claims**

1. A process for the preparation of *alpha*-cyanobenzyl esters of general formula (I):

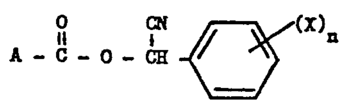

(I)

by reacting a carboxylic acid halide of general formula (II):

A—CO-halide (II)

with an *alpha*-cyanobenzyl alcohol of general formula (III):

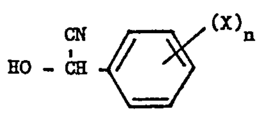

(III)

in the presence of an inert solvent, wherein in the general formulae A is an optionally-substituted aralkyl, alkyl or cycloalkyl group containing up to 24 carbon atoms; X is an alkyl, alkenyl, aralkyl or aryloxy group containing up to 10 carbon atoms; n is an integer of 1 to 3; and halide is bromine or chlorine, characterized in that the reaction is carried out in the presence of a Lewis acid catalyst.

2. A process according to claim 1, characterized in that the Lewis acid catalyst is an inorganic compound derived from titanium, tin, antimony, tantalum, rhenium, iron or zinc.

3. A process according to claim 3, characterized in that the inorganic compound is an inorganic halide or cyanide.

4. A process according to claim 2 or 3, characterized in that the Lewis acid catalyst is a zinc halide.

5. A process according to claim 4, characterized in that the zinc halide is zinc chloride.

6. A process according to any one of the preceding claims, characterized in that the *alpha*-cyanobenzyl ester is an *alpha*-cyano-3-phenoxybenzyl alpha-isopropylphenylacetate.

## Revendications

1. Procédé pour la préparation d'esters *alpha*-cyanobenzyliques de formule générale (I):

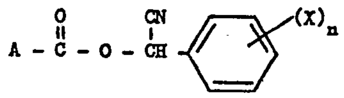

(I)

en faisant réagir un halogénure d'acide carboxylique de formule générale (II):

A—CO-halogénure (II)

avec un alcool alpha-cyanobenzylique de formule générale (III):

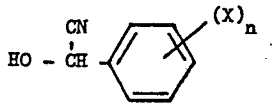

(III)

en présence d'un solvant inerte, où dans les formules générales A est un groupe aralcoyle, alcoyle ou cycloalcoyle éventuellement substitué contenant jusqu'à 24 atomes de carbone; X est un groupe alcoyle, alcényle, aralcoyle ou aryloxy contenant jusqu'à 10 atomes de carbone; n est un nombre entier de 1 à 3 et "halogénure" veut dire chlorure ou bromure, caractérisé en ce que la réaction est conduite en présence d'un catalyseur acide de Lewis.

2. Procédé selon la revendication 1, caractérisé en ce que le catalyseur acide de Lewis est un composé inorganique dérivé du titane, de l'étain, de l'antimoine, du tantale, du rhénium, du fer ou du zinc.

3. Procédé selon la revendication 2, caractérisé en ce que le composé inorganique est un halogénure ou cyanure inorganique.

4. Procédé selon l'une des revendications 2 ou 3, caractérisé en ce que le catalyseur acide de Lewis est un halogénure de zinc.

5. Procédé selon la revendication 4, caractérisé en ce que l'halogénure de zinc est du chlorure de zinc.

6. Procédé selon l'une quelconque des revendications précédentes, caractérisé en ce que l'ester *alpha*-cyanobenzylique est l'alpha-isopropylphénylacétate d'*alpha*-cyano-3-phénoxybenzylique.

## Patentansprüche

1. Verfahren zur Herstellung von $\alpha$-Cyanobenzylestern der allgemeinen Formel (I):

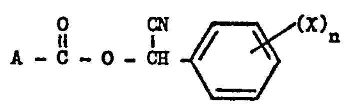

(I)

durch Umsetzen eines Carbonsäurehalogenids der allgemeinen Formel (II):

A—CO-Halogenid (II)

mit einem $\alpha$-Cyanobenzylalkohol der allgemeinen Formel (III):

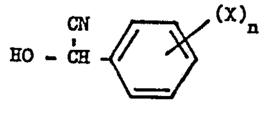

(III)

in Gegenwart eines inerten Lösungsmittels, wobei in den allgemeinen Formeln A eine gegebenenfalls substituierte Aralkyl-, Alkyl- oder Cycloalkylgruppe mit bis zu 24 Kohlenstoffatomen ist, X eine Alkyl-, Alkenyl-, Aralkyl- oder Aryloxygruppe mit bis zu 10 Kohlenstoffatomen bedeutet, n eine ganze Zahl von 1 bis 3 ist und Halogenid Brom oder Chlor bedeutet, dadurch, gekennzeichnet, dass die Reaktion in Gegenwart einer Lewis-Säure als Katalysator ausgeführt wird.

2. Verfahren nach Anspruch 1, dadurch gekennzeichnet, dass der Lewis-Säure-Katalysator eine

anorganische Verbindung von Titan, Zinn, Antimon, Tantal, Rhenium, Eisen oder Zink ist.

3. Verfahren nach Anspruch 2, dadurch gekennzeichnet, dass die anorganische Verbindung ein anorganisches Halogenid oder Cyanid ist.

4. Verfahren nach Anspruch 2 oder 3, dadurch gekennzeichnet, dass der Lewis-Säure-Katalysator eine Zinkhalogenid ist.

5. Verfahren nach Anspruch 4, dadurch gekennzeichnet, dass das Zinkhalogenid Zinkchlorid ist.

6. Verfahren nach einem der vorangehenden Ansprüche, dadurch gekennzeichnet, dass der $\alpha$-Cyanobenzylester ein $\alpha$-Cyano-3-phenoxybenzyl-$\alpha$-isopropylphenylacetat ist.